# EUROPEAN PATENT APPLICATION

(11) **EP 1 166 779 A2**
(43) Date of publication of application: **02.01.2002**
(21) Application number: 01114284.1
(22) Date of filing: 13.06.2001
(51) Int. Cl.: A61K 31/18, A61K 47/48, A61P 17/00, A61P 17/06

(54) **The use of nimesulide in the treatment of dermatological diseases**

(30) Priority: 20.06.2000 IT MI001378
(71) Applicant: Helsinn Healthcare S.A., 6912 Pazzallo-Lugano (CH)
(72) Inventor: Villa, Giuliana, 6912 Pazzallo (CH); Macchi, Fabio, 6912 Pazzallo (CH); Bevilacqua, Maurizio, 6912 Pazzallo (CH)
(74) Representative: Minoja, Fabrizio, Dr.

(57) **Abstract**

The use of Nimesulide or of a physiologically equivalent form thereof for the preparation of medicaments for the treatment of dermatological diseases.

## Description

The present invention relates to the use of Nimesulide, or of a physiologically equivalent form thereof, for the preparation of topical medicaments for the treatment of dermatological diseases characterized by accumulation of polymorphonuclear leukocytes.

These diseases, besides psoriasis in the strict sense of the word, include the following:
Pustular psoriasis: characterized by accumulation of polymorphonuclear leukocytes (in the following "polymorphonucleates") with perforation channels opening at the surface of dermis, which appear as amicrobic pustules, that may be generalized or localized to the palms and soles. The typical psoriatic lesions may often be absent.
Gangrenous pyoderma: accumulation of polymorphonucleates.
Capillitium erosive dermatitis: characterized by accumulation of leukocytes and neutrophils in the dermis, symptoms are pustules, erosions, crusts and scaling of the skin. This disease is progressive and leads to cicatritial alopecia.
Dick & Syme syndrome: characterized by annular perforating granuloma, episcleritis and eosinophilia. The disease is usually localized on the hands, and it appears with subcutaneous nodules and lesions discharging collagen in the form of a viscous fluid, which dries forming yellow crusts. In this case also, accumulation of polymorphonucleates and eosinophilia are present.

All of these diseases (which hereinafter will be referred to as "diseases") are generally characterized by accumulation of polymorphonucleates in the dermis (sterile abscess) with perforation channels which open on the dermis. No medicaments ensuring total eradication of these diseases, in particular of pustular psoriasis, exist at present, on the contrary, few effective medicaments are available.

The simplest forms are usually treated with lubricant creams based on hydrogenated vegetable oils, white petrolatum, crude coal tar, salicylic acid, zinc sulphate, anthranyl compounds (dithranol), optionally associated with natural or artificial UV irradiation.

These treatments are however poorly effective in the case of serious, generalized forms.

Non steroid anti-inflammatory drugs are quite unsuitable as the cause exacerbation of the skin lesions, in particular of the pustular ones.

Methotrexate, a known antimetabolite, is very effective, but its use should be restricted to the most severe cases, due to its toxic side effects, such as myelosuppression, anaemia, leukopaenia, hepatic damage and the like.

Corticosteroids cannot be used systemically as they induce serious exacerbation of the "diseases", in addition to the known side effects. They are effective topically, but they can induce iatrogenic side effects (telangiectasias, atrophy). Furthermore, the administration thereof on large areas of the body, especially under occlusion, is not recommended as this can cause severe systemic effects as well as aggravating the "diseases".

PUVA, a treatment based on methoxalene followed by irradiation with ultraviolet radiation, often induces remission of even severe forms, but it can induce serious side effects, such as epitheliomas.

Etretinate or isotretinoin are very effective, but they induce protracted teratogenicity (for at least two years after interruption of the administration).

It is therefore evident that a topically effective medicament, which induces none of the above mentioned side effects, is strongly needed.

Nimesulide is a non-steroidal anti-inflammatory drug (NSAD) used for some time in the treatment of various inflammatory and painful conditions. Nimesulide acts through the selective inhibition of prostaglandin biosynthesis.

It has now surprisingly been found that Nimesulide, when administered topically/transdermally, acts effectively on the lesions caused by the "diseases", remarkably improving the skin lesions.

Recent evidence indicated an alteration of the extra-vasation mechanisms of neutrophil leukocytes in the aetiology of both skin manifestations and synovitis/psoriatic arthritis. The cause might be a defect of the adhesion mechanisms of neutrophils on venule endothelium, in its turn caused by scaling of "adhesines" endothelial cells.

The suggested action mechanism would consist in an inhibitory effect on:
- neutrophil leukocytes activation,
- production of oxidizers and other inflammatory mediators,
- inhibition of neutrophil leukocytes extravasation.

Therefore the present invention relates to the use of Nimesulide or a physiologically equivalent form thereof in the treatment of the "diseases".

More particularly, the present invention relates to the use of Nimesulide or of a physiologically equivalent form thereof for the preparation of pharmaceutical compositions for the treatment of the "diseases".

According to the invention, Nimesulide or of a physiologically equivalent form thereof will be administered on the surface of the affected skin once or twice a day, through the topical/transdermal routes, in the form of a formulation containing 1 to 20 % by weight of active ingredient. In particular, in the case of a 3% gel, daily dosages will range from 100 to 500 mg of active principle.

Examples of physiologically equivalent forms of Nimesulide comprise salts, such as those disclosed in EP-A-937709, complexes with cyclodextrins, (WO94/02177) or other forms, which are converted into Nimesulide or into its active metabolites after administration.

Nimesulide or the equivalent forms thereof will be suitably formulated in the form of creams, gel, ointments, ointments, solutions, suspensions, powders, plasters and the like. Examples of preferred formulations are those disclosed in WO 96/11002, EP-A-1007001, WO 98/37879, herein incorporated for reference.

Double blind clinical studies were carried out on patients suffering from pustular psoriasis, gangrenous pyoderma, capillitium erosive dermatitis and Dick & Syme syndrome. Patients were treated with Nimesulide, at a dosage of 200 mg/day for 3 weeks, followed by 100 mg/day for three weeks.

At the end of the treatment, the following parameters were evaluated:
- number of the skin lesions
- extent of the skin lesions
- relief from pain at the nodule sites
- subjective evaluation.

Nimesulide was found statistically effective in the treatment of the "diseases", as it induced the regression of the skin lesions in terms of extent and number, and the almost complete disappearance of pain localized at the nodules. In some cases the treatment induced the complete remission of the "disease". Furthermore, no side effects were observed.

## Claims

1. The use of Nimesulide or a physiologically equivalent form thereof for the preparation of a medicament for the treatment of dermatological diseases **characterized by** accumulation of polymorphonuclear leukocytes.

2. The use as claimed in claim 1, in which the dermatological diseases **characterized by** accumulation of polymorphonuclear leukocytes are: pustular psoriasis, gangrenous pyoderma, capillitium erosive dermatitis, Dick & Syme syndrome.

3. The use as claimed in claim 1 or 2, in which the physiologically equivalent form of Nimesulide is a salt thereof or a complex with cyclodextrins.

4. The use as claimed in claims 1 - 3, in which Nimesulide or the physiologically equivalent form thereof are administered in the form of creams, gel, ointments, ointments, solutions, suspensions, powders, plasters and the like.
